# EUROPEAN PATENT SPECIFICATION

(11) **EP 3 160 337 B1**
(45) Date of publication and mention of the grant of the patent: **06.04.2022**
(21) Application number: 15736679.0
(22) Date of filing: 26.06.2015
(51) Int. Cl.: A61B 5/024, A61B 5/1455

(54) **BODY WORN MEASUREMENT DEVICE**
AM KÖRPER GETRAGENE MESSVORRICHTUNG
DISPOSITIF DE MESURE PORTÉ SUR LE CORPS

(30) Priority: 30.06.2014 NL 2013091; 16.04.2015 NL 1041276
(43) Date of publication of application: 03.05.2017
(73) Proprietor: Scint B.V., 5056 SM Berkel-Enschot (NL)
(72) Inventor: SCHILTHUIZEN, Stephanus Franciscus, 5056 SM Berkel-Enschot (NL)
(74) Representative: V.O.
(86) International application number: PCT/NL2015/050468
(87) International publication number: WO 2016/003269

(56) References cited:
- JP-A- 2013 063 203
- US-A1- 2003 036 685
- US-A1- 2007 055 163
- US-A1- 2009 018 453
- US-A1- 2011 105 918
- US-A1- 2012 229 270
- US-A1- 2013 253 332
- US-A1- 2013 296 714

## Description

### TECHNICAL FIELD

The invention relates to a measurement device worn on the body and a method for performing a measurement using such a device.

### BACKGROUND

Body worn devices for measuring a (signal representative of a) physiological parameter of a user are known in the art, see e.g. us 2013/253332 A1, US 2013/296714 A1. Unfortunately, such devices may be unreliable, and sometimes measure a value of certain physiological parameters in rest positions and under limited and more severe movement that does not correspond to the actual value of that parameter of the user. These problems can be exacerbated while attempting to measure a physiological parameter of a user during exercise and/or during movements of the skin, body and arms in general. The effect of the exercises, skin, body fluids and body and body part movements on the measurements is for many know devices today that these are not reliable and are largely influenced by (motion) artefacts and inaccurate measurements.

### SUMMARY OF THE INVENTION

It is an object of the invention to provide a more reliable device and method for measuring a signal representative of a physiological parameter of a user, at rest, under movement and during exercise and providing more comfortable solutions for carrying the device on the body on the skin for a longer time.

Thereto, according to the invention a method for measuring a signal representative of a physiological parameter of a user is provided as defined in appended independent claim 1.

Further, according to the invention a body worn device for measuring a signal representative of a physiological parameter value is provided. The device according to the invention is defined in appended independent claim 10.

A method for measuring a health status of a user is provided comprising the steps of: providing a device worn on a wrist of the user, wherein the device includes, a securing band or strap, at least one light source having a light emitting surface and at least one light receiver having a light receiving surface. The at least one light source and the at least one light receiver are arranged on the securing band of the device such that when worn, the at least one light emitting surface and the at least one light receiving surface substantially abut against the palmar side of the wrist of the user, e.g. over one or both of the main arteries (radial and ulnar artery) within the wrist, or on a higher position on the forearm or upper arm or on the finger or another position on the wrist. The method includes performing a measurement session including; emitting light in a wavelength range by the at least one light source for a predetermined amount of time; producing a received light signal by the at least one light receiver corresponding to a received intensity of light received at the at least one light receiver; defining an amplitude envelope control band having an envelope upper amplitude value and an envelope lower amplitude value, and controlling the amplitude of the received light signal to be within the amplitude envelope control band by controlling a force pressing the at least one light emitting surface and/or at least one light receiving surface in a direction substantially perpendicular to the palmar side of the user's wrist. The controlling can include maintaining the amplitude of the received light signal within the amplitude envelope control band.

The amplitude envelope control band defines a desired amplitude range for the received light signal taking into account the expected health status of the user and/or any motion artefacts due to the movement of the body and limbs of the user and/or the movement of device on the skin and/or the flow of blood in the arteries and other blood vessels due to the physical movement and position of limbs. If the amplitude of the received light signal is outside of, or close to the edge of, the amplitude envelope control band, the amplitude of the received light signal is controlled to be within the amplitude envelope control band by controlling the force pressing the at least one light emitting surface and/or at least one light receiving surface in a direction substantially perpendicular to the palmar side of the user's wrist. In this way the radiative flux into the palmar side of the wrist and/or the radiative flux out of the palmar side of the wrist is controlled.

It will be appreciated that controlling the amplitude of the received light signal to be within the amplitude envelope control band implies making a best effort at maintaining the amplitude of the received light signal in the amplitude envelope. It is conceivable that the amplitude of the received light signal departs from the amplitude envelope control band while performing the method. For instance a temporary overshoot is known in control mechanisms. Controlling the force pressing the at least one light emitting surface and/or at least one light receiving surface brings the amplitude of the received light signal back within the amplitude envelope control band.

Controlling the received light signal to be within the amplitude envelope control band results in better and more reliable measurements derived from the received light signal.

Additionally, it will be appreciated that a health status can include a general fitness, heart rate measurement and derivates thereof such as heart rate variability value which is directly related to levels of stress, heart rate recovery rate value which is related to a general fitness or physical health condition, the oxygen saturation rate within the blood which can be measured by using two light sources with different wavelengths, the respiration rate on basis of the periodic fluctuation of the heart rate signal, the CO₂ saturation rate in venous blood, arteries and in other blood vessels, the blood pressure and/or a compliance to an exercise schedule of a user.

Additionally it will be appreciated that the controlling mechanism can also be combined with another type of sensor, such as an electrically conductive patches, to measure ECG, EEG or EMG signals. An embodiment of the invention can be a band around the upper arm with two integrated ECG electrodes which can pressed on the skin more when the derived electro potential signal is too weak or too much distorted by skin and body movements. This can also be in the shape of an ECG-chest strap or ECG-shirt.

Optionally, the controlling includes decreasing the force when the amplitude of the received light signal exceeds the envelope upper amplitude value and increasing the force when the amplitude of the received light signal is below the envelope lower amplitude value. It has been found that when the amplitude of the received light signal exceeds the envelope upper amplitude value, the radiative flux into and/or out of the palmar side of the wrist should be decreased. This can be achieved by decreasing the force pressing on the at least one light emitting surface and/or the at least one light receiving surface. Similarly, it has been found that when the amplitude of the received light signal is below the envelope lower amplitude value, the radiative flux into and/or out of the palmar side of the wrist should be increased. This can be achieved by increasing the force pressing on the at least one light emitting surface and/or the at least one light receiving surface.

Optionally, the method further includes controlling the amplitude of the received light signal to be within the amplitude envelope control band by controlling an emitted intensity of light emitted by the at least one light source. It is conceivable that in order to better control the amplitude of the received light signal to be within the amplitude envelope control band, that in additional to the pressing force, the emitted intensity of light emitted by the at least one light source is controlled. The emitted intensity of light emitted by the at least one light source can be measured as radiant emittance or radiant exitance measured as power per unit area radiated by a surface.

Optionally, the method includes determining the amplitude envelope control band by measuring the received light signal during a predetermined period, and determining the envelope upper amplitude value and envelope lower amplitude value on the basis of the measured received light signal during the predetermined period. It is also possible that the amplitude envelope control band is predetermined, or selected from a series of predetermined amplitude control bands.

Optionally, the step of determining the amplitude envelope control band is performed at the start of the measurement session. In this way the amplitude envelope control band corresponds to the conditions present at the beginning of the measurement session.

According to the invention, the step of determining the amplitude envelope control band is repeated at a plurality of moments during the measurement session so as to set a new amplitude envelope control band during the measurement session. In this way the amplitude control band is set to correspond to changing conditions during the measurement session. Optionally, the step of determining the amplitude envelope control band is performed continuously during the measurement session.

Optionally, the method further includes determining a relationship between the pressing force and the received light intensity depending on the anatomy, skin type, position of arteries etc. This relationship may be used in the step or steps of controlling the amplitude of the received light signal to be within the amplitude envelope control band. The relationship may be used as a transfer function for determining a required amount of change in the pressing force to achieve a desired amount of change in the amplitude of the received light signal.

Optionally, the method further includes applying a step in the force pressing the at least one light emitting surface and/or at least one light receiving surface in a direction substantially perpendicular to the palmar side of the user's wrist and determining a step response of the received light intensity. The determined step response may be used in the step of controlling the amplitude of the received light signal to be within the amplitude envelope control band.

Optionally, the method further includes determining whether the controlling mechanism should be activated based upon the received light signal, based upon the received light signal and one or more additional sensor signals (pressure sensor and/or accelerometer sensors and/or additional light signals from a different wavelength received by the light receiver etc.) or based on a received light signal from an addition light source with a wavelength which is not able to penetrate the skin deeply and which will show more clearly movement of light source and light receiver on the skin (e.g. blue and/or green LED-light).

According to another aspect the device includes a plurality of light emitting and light receiving surfaces, which can be used to perform measurements of physiological parameters such as heart rate, heart rate variability and heart rate recovery, an other ones such as oxygen saturation (SpO2), respiration rates, CO₂ values and a separate set or sets of light emitting surfaces and receivers with a different wavelength than the ones used for illuminating of the blood vessels and the above mentioned measurements of parameters, which separate set or sets of light emitting surfaces and receivers can be used for the specific detection of motion artefacts and movement. These specific light emitters emit light having either a low penetration depth in the skin (e.g. 400-550 nm) and/or a high water absorption rate (e.g. 1100-1400 nm). The parallel detection of small or bigger motion artefacts can be used in algorithms and software processing to detect at all times the heart rate of the wearer of the device under movement from and within the received light signals. These light emitters and if necessary receivers can be covered with polarizers to filter the light emitted and received in such a way and under such angle that specific movement can be detected. Therefore a polarisation filter can be placed on the light source resulting in a light beam polarised in one direction. The light reflected on the skin rotates the polarisation direction of the light by ninety degrees. By placing a polarisation filter on the optical sensor turned by ninety degrees in reference to the polarisation filter at the light source, the sensor is more sensitive to the reflected light and less to the scattered light through the tissue. This embodiment will enable detection of heart rates under medium and more severe movement of body, arm and/or the device on the arm, due to the ability to distinguish in the signals between movement or motion artefacts and heart rate values.

According to another two aspects the device contains two solutions to limit or omit the influences of circumferential, ambient light. The first solution hereto makes use of flexible deformable non-transparent skirt around the light emitting and light receiving surfaces, touching the skin and following the contour of the skin and blocking penetration of all ambient light. The second solution is the use of modulation of light in the system to subtract possible ambient light values from the measurements being made by the light receiving surfaces.

According to another aspect the parallel sensing of heart rate values and high motion artefacts and movement with specific light emitting and light receiving surfaces will make it possible to asses physical exercise, movement and activity, if necessary combined with accelerometer data, GPS data, tri-angulation GSM positioning data and other available means.

Optionally, the method further includes determining the amount of pressing of the controlling mechanism based upon the received light signal, based upon the received light signal and one or more additional sensor signals (pressure sensor, accelerometer sensors, additional light signals from a different wavelength received by the light receiver etc.) or based on a received light signal from an addition light source with a wavelength which is not able to penetrate the skin deeply and which will show more clearly movement of light source and light receiver on the skin (e.g. blue and/or green LED-light).

Optionally, the method further includes determining whether the controlling mechanism should be activated, and optionally with what displacement and force, based upon the detection of motion artefacts. This detection of motion artefacts can e.g. include the detection of variation of the received amplitude envelope picked up by a light receiving surface part. The detection of motion artefacts can also be performed using the above mentioned additional sensors, such as an optical light receiving sensor, receiving light of light emitter surface with a wavelength which doesn't penetrate the skin easily reflected on the skin, a pressure sensor detecting the contact pressure (and/or force) between the light emitting and receiving surfaces and the skin, or an accelerometer or combinations of some or all of these additional or already present sensors.

Optionally, the method further includes determining the user's heart rate and/or related parameters such as heart rate variability ('HRV") and heart rate recovery rate ("HRR") and oxygen saturation ("SpO2"), possibly the CO2 concentration or saturation rate and the respiration rate on the basis of the received light signal based on time and/or frequency domain analysis, and optionally on the basis of a value of a control signal of the control unit for controlling the force and/or emitted intensity. The high frequency component of the received light signal may also be used.

Optionally, the method further includes performing a first measurement session wherein the light emitted by the at least one light source is in a first wavelength range and performing a second measurement session wherein the light emitted by the at least one light source is in a second wavelength range. Using light in a first wavelength range followed by light in a second wavelength range allows for additional health indicators to be determined.

Optionally, the step of performing a measurement session further includes a step of calculating a perfusion index value on the basis of the received light signals of two light sources or two light wavelengths or frequencies of e.g. 600-660 nm and 880-940 nm. Optionally, the method further includes determining the user's saturation of peripheral oxygen level, SpO2, on the basis of a ratio of the perfusion index value calculated in the first measurement session and the perfusion index value calculated in the second measurement session, and optionally on the basis of a value of a control signal of the control unit for controlling the force and/or emitted intensity. Here the oxygen saturation of the user's blood and changes in the blood volume in the skin are monitored. The changing absorbance at each of the wavelengths is measured allowing the absorbance due to the pulsing of the blood to be determined. The wrist worn device can act as a plethysmograph, and a photoplethysmogram may be produced and displayed.

The perfusion index valve can be calculated on the basis of a ratio of a high frequency component and a low frequency component of the received light signals.

Optionally, the actuator includes one of a spool and magnet, for example a solenoid or a voice coil, an inflatable body, a piezoelectric actuator, a linear motor, a gear, a conductive polymer or textile structure able to alter it's dimensions in radial, length and/or width directions when a current is applied or a combination of one or more of these elements.

Optionally, the actuator includes two or more spools and magnets per set of light source and light receiver, for example a solenoid or a voice coil, an inflatable body, a piezoelectric actuator, a linear motor, a gear, a conductive polymer or textile structure able to alter it's dimensions in radial, length and/or width directions when a current is applied, which make it possible to control the pressing force in two or three orthogonal directions and not only perpendicular to the wrist.

When the controlling mechanism per set of light sources and light receiver consist of a spool and magnet the magnetic fields of these element should be controlled and directed in such a way that the magnets will not be attracted to each other and that the controller doesn't influence the received light signal. This can e.g. be done e.g. via the use of metal alloys with a high magnetic permeability. Elements of these metals can cover top and bottom of the spool, which has the magnet inside or cover also the outside cylindrical part of the spool.

Another option to overcome this attraction of magnets is to put the magnet in a housing, which has a rigid top and bottom part, which the magnet can't pass. On top of the magnet a cylindrical, rigid part (e.g. with a diameter smaller than the magnet) can be positioned which slides up and down inside the electrical spool together with magnet during actuation and performs the actual pressing on the skin.

Optionally, the actuator includes a cylindrical magnet with an inner hole in which a spool moves up and down. By this the dimensions of the actuator can be minimized while maintaining of the force to be exerted. Hence, the magnets of each set will not be attracted to each other, while the magnet itself can be mounted and fixed in a polymer casing and is not the moving part. The smaller diameter of this embodiment will enable an array of light emitting and light receiving surfaces to be actuated, with reduced power consumption, close to each other in a desired spot at the inner side of the wrist or the arm.

Optionally, the actuator includes a ring or raised area above the housing of the hollow cylindrical spool and inner magnet or cylindrical hollow magnet and inner spool, which allows the light emitter and light receiver surfaces which are initially pressed into the inner, lower area of this ring or raised area after putting on the band/strap of the wrist worn device, to be pushed even further into the skin if necessary or to be released slightly in pressing force to the skin depending on the movement direction of the actuator to achieve the desired signal quality. This ring or raised area, which can have a ring shape which gradually increases from the ground (side of band) to the upper level (contact side with skin) and which can be manufactured of a rigid or slightly deformable material (e.g. rubber or polymer foam, can have a 3-dimensional spacer fabric, deformable by design), can be covered by the inner textile part of the band/strap, which has a small hole in the center area above the ring, to allow the light emitting and light receiving surfaces to be pushed slight above or through this hole to enable direct skin contact which is beneficial for an effective light coupling.

The light emitting and light receiving surfaces are preferably positioned close to each other or mounted as bare-die chips on a small flexible or rigid printed circuit board to minimize the required controlling force of the actuator and by this the actuator dimensions and the use of electric power.

The light emitting surfaces are preferably arranged around one or two light receiving surfaces in a position close to each other to maximize the light coupling and light receiving and to minimize the required controlling force of the actuator and by this the actuator dimensions.

The light emitting and light receiving surfaces are preferably covered or moulded into a spherical or half cylindrical shaped polymer material which should be transparent above the light emitting and light receiving surfaces and can be, if necessary for the application, partly less transparent via the use of a coloured pigment additive or completely or partly non-transparent, e.g., grey or black, in the areas between the light emitter and light receiver.

According to an aspect, the light emitting surface(s) and the light receiving surface(s) can be separated by a deformable non-transparent light blocker. Each light emitting surface may be circumscribed by a deformable light blocking element made from a compressible material, each light receiving surface may be circumscribed by a light blocking element made from a compressible material. The light blocker may e.g. be a black or grey plastic material. This helps in preventing direct radiation from a light emitting surface entering a light receiving surface, without travelling through the skin of the user first. The compressible light blocking material will allow an appropriate pressing force on the skin, which is not too high to cause discomfort and is still high enough to enable a good signal without interference of ambient, circumferential, surrounding light, which can be blocked extra and initially by the partly compressed light blocker touching the skin around the area where light is being emitted in the skin and reflected light is being collected and measured by the light receiving surface or sensor.

According to an aspect , the device further also can include a deformable non-transparent plastic, rubber, polymer or metal element or a combination of these elements, projecting towards the side of the securing band facing the wrist or forearm, such that the light emitting surface and/or the light receiving surface are shielded from all sides between skin and the device itself from ambient, surrounding light and are if necessary pressed with this deformable part into the skin of the wrist. This deformable non-transparent element which can deform due to its material properties and/or specific shape and will block the surrounding, ambient or environmental light, allows comfortable wearing of the wrist worn device without too high pressures causing discomfort. It completely shields the light emitting and light receiving surfaces from surrounding natural and or artificial light, it provides due to it's compression and higher friction characteristics a stable interface to the skin, reducing motion and other artefacts and enabling stable assessment of heart rate, SpO2 and other physiological signals.

Optionally the actuator can also be used to position and press the light emitting and light receiving surface via an actuator movement more tightly or more loosely in the skin to achieve an optimum signal and to maintain this position during the measurement session.

Optionally, the at least one light source includes a plurality of light sources and at least one light receiver includes a plurality of light receivers, e.g. arranged around (part of) the circumference of the arm or wrist, perpendicular to the arm and/or parallel to the arm and/or in other directions.

According to another aspect a method of eliminating the influences of the environmental, surrounding or ambient light in the measurements is realized using a modulation scheme. The light emitting sources are alternately driven in a maximal and minimal light intensity. A light source i.e. LED, is driven by a current (or voltage) source, and the light of the illuminated tissue is measured by a light receiving surface, the sensor i.e. photodiode or PD as mentioned before. The induced current of the sensor is amplified and digitized using an Analog to Digital Convertor (ADC) in the hardware of the wrist worn device.

The light source is driven to a maximal or a certain high intensity resulting in a maximal or a certain high ADC output. The light emitting source (LED) is driven to a minimal or a certain low intensity resulting in a minimal or a certain low ADC output. Those two readings are compared with each other (e.g. subtracted from each other or the one divided by the other) in the firmware or embedded software resulting in the final measurement reading. In this way the environmental light which is present as well in the high intensity as in the low intensity measurement is being eliminated. The alternating driving electrical current for the light emitting sources in this method can be a square block signal with a certain offset and amplitude. However, other waveforms can also be used. The maximal and minimal value of the current is automatically calibrated before starting a measurement. A light source electrical current value or setting can be adjusted in such a way that if the sensor reading exceeds for instance 10% of the ADC range, that electrical current value is then used as the minimal value of the light source current. A light source electrical current value can then be adjusted in such a way that if the sensor reading exceeds for instance 90% of the ADC range, that electrical current value is then used as the maximal value of the light source current.

A comparable modulation-demodulation scheme can be employed with a light source driven by a periodic electrical current signal like a sinusoidal signal. The demodulation scheme at the sensor side can then be realized by a simple multiplication of the measured signal by the same sinus signal used for the modulation followed by a low pass filtering. The maximal value of the sinusoidal current signal to the light source has to be adjusted to induce a maximal reading at the sensor side, and the minimal value of the sinusoidal current signal to the light source has to be adjusted to induce a minimal reading at the sensor side.

The controlling of the signal can also be achieved via the use of a set with at least three or more light emitting surfaces per light receiving surface/sensor, positioned and pressed into the skin area directly above the arteries in the wrist, e.g. the radial artery on the thumb side and the ulnar artery on the smallest fingers side or on another spot of the forearm.

Providing a plurality of light sources and a plurality of light receivers allows for different possibilities of emitting light. Furthermore, with a plurality of receivers the received light signal can be produced in different ways.

Optionally, the step of producing the received light signal at the at least one light receiver includes averaging the received light signals produced at a subset of the plurality of light receivers. Through averaging a more reliable received light signal can be produced.

Optionally, the step of producing the received light signal at the at least one light receiver includes selecting an optimum received light signal produced at one of the light receivers. In this way a received light signal is produced at more than one of the light receivers, and the optimum received light signal is selected. The optimum received light signal may e.g. be the received light signal having the largest amplitude and/or level. Optionally, the optimum received light signal is selected on the basis of a signal to noise ratio. The optimum received light signal may e.g. have the largest signal to noise ratio, meaning a distinguishing AC (heart rate pulsation) part above the DC part of the received light.

Optionally, the step of producing the received light signal at the at least one light receiver includes selecting the best and second best received signal produced at two of the light receivers. Additionally, the best and second best received signal may be combined for example by averaging. Herein the best signals may be the signals having the highest level, amplitude and/or signal to noise ratio.

Optionally, the method further includes detecting a movement of the user, and or the device on the skin on the basis of the received light signals, and optionally on the basis of a value of a control signal of the control unit for controlling the force and/or emitted intensity. Detecting a movement of the user and or device on the skin can be used to determine the amount or level of artefacts influencing the received signal, to determine if a user is following a prescribed exercise regime, and/or to determine if a user is moving enough to achieve a given health status.

Optionally, the step of determining the amplitude envelope of the received light signal includes filtering the received light signal, or the square of the received light signal, with at least one of a low pass filter having a cut-off frequency at least less than a highest expected heart rate, preferably substantially equal to 4 Hz, and a bandpass filter having a pass-band defined by the lowest expected heart rate and a highest expected heart rate, preferably substantially equal to 0.7 Hz and 4 Hz.

In this way artefacts arising in the received light signal that correspond to a heart rate outside of the range from the lowest expected heart rate to the highest expected heart rate can removed. The lowest expected heart rate and highest expected heart rate may be predefined. Alternatively, the lowest expected heart rate and the highest expected heart rate may be variable, and may be determined on the basis of the user's detected movement, a time of day, the user's age, and/or the user's sex. The expected heart rate may also be deduced from previous measurements of the user's heart rate by the device.

Optionally, the step of determining the relationship between the pressing force and the received light intensity is determined for a range of forces exerted on the at least one light source and at least one light receiver. In this way the step of maintaining the received light signal in the amplitude envelope control band is facilitated. Optionally, the relationship is stored, for example in a look-up table.

Optionally, the method further comprises a step of selecting the at least one light source from the plurality of light sources and selecting the at least one light receiver from the plurality of light receivers, wherein the step of selecting comprises; for each light source of the plurality of light sources emitting light in a wavelength range for a predetermined amount of time; producing at each light receiver a received light signal corresponding to the light received at that light receiver individually from each light source; separating each received light signal into a high frequency component and a low frequency component; calculating for each received light signal a perfusion index value on the basis of a ratio of the high frequency component and the low frequency component, and selecting a light source and light receiver combination having the highest perfusion index value as the at least one light source and the at least one light receiver.

It has been shown that selecting the light source and light receiver combination having the highest perfusion index value provides an improved received light signal. Different filtering possibilities exist for separating each received light signal into a high frequency component and a low frequency component. The cut-off values frequencies may be based on the lowest expected heart rate and/or the highest expected heart rate.

Optionally, the method includes selecting a subset of light sources of the plurality of light sources and a subset of light receivers of the plurality of light receivers having the highest perfusion index values as the at least one light source and the at least one light receiver. In this way methods of producing the received light signal on the basis of more than one light source and light receiver combination can also be performed.

Optionally, the step of selecting is performed for a first wavelength range and a second wavelength range, and optionally for a third and optionally fourth wavelength range. It is conceivable that different light source and light receiver combinations are ideal for the first wavelength range and the second wavelength range. It can be advantageous to determine the light source and light receiver combination for each range independently.

Using light in a first wavelength range followed by light in a second wavelength range etc. allows for additional health indicators to be determined and/or to determine the heart rate parameter via two measurements. The amount of different wavelengths to be used can be one, two, three or more depending on the desired accuracy, artefact compensation measures, redundancy and additional health indicators to be measured.

The improvement of the signal can also be achieved via the use of at least two sets of light emitting surfaces and light receiving surfaces, one set positioned and pressed into the skin area on the lower side of the wrist directly on top of the radial artery and or ulnar artery and one set on the upper side of the wrist. Here also smaller blood vessels are present and can be measured with light emitting and light reflection measurement techniques as described before.

The improvement of the signal can also be achieved via the use of at least one set of light emitting surfaces and light receiving surfaces, positioned on the upper side of the wrist and which can be pressed with a larger or smaller force into the skin. It will be appreciated that the method can include controlling a force pressing a first sensor in a direction substantially perpendicular to the body of the user at the upper side of the wrist and controlling a force pressing the a second sensor in a direction substantially perpendicular to the body of the user at the lower side of the wrist.

The improving of the signal can also be achieved via the use of the compressible light blocking shielding around the set or sets of light emitting and light receiving surfaces. The improving of the signal can also be achieved via the use of modulation scheme for the LEDs and PDs as described above.

Optionally, the method further comprises a step of displaying on a display associated with the wrist worn device an instruction instructing and advising the user to perform a predetermined exercise; performing a measurement session while the user performs the predetermined exercise; and storing a result of the measurement session, wherein the result preferably includes the user's heart rate and/or saturation of peripheral oxygen level and/or ability to recover the user's heart rate at a normal, non-exercising level, for example heart rate recovery.

In this way, an indication of the user's health can be determined while performing a predetermined exercise. The display may be included in the wrist worn device. Additionally, or alternatively, the display may be included in an external device, such as a smart phone device, that is wired or wirelessly communicatively connected with the wrist worn device.

Optionally, the method further comprises, measuring the general fitness of the user on the basis of the measurement session and/or previous measurement sessions; displaying an indication of the general fitness and/or health of the user. In this way, the user and/or a health advisor can quickly determine an indication of the user's general fitness.

Preferably a graphical representation of a person having a general fitness corresponding to the general fitness of the user is displayed. For example if it is determined that the general fitness of the user is poor, the displayed graphical representation of the person may have his hands on his knees and be breathe heavily. On the other hand if the general fitness of the user is good, the displayed graphical representation of the person may be performing jumping jacks, or be jogging in place. Or on the other hand if the oxygen saturation value and or heart rate recovery rate of the user is good, the displayed graphical presentation of the person may be moving faster than if the oxygen saturation value and or heart rate recovery rate of the user is less good, giving an indication of a lower general fitness or deteriorating health condition.

Optionally, the graphical representation moves and is coloured corresponding to the general fitness of the user and/or compliance to the exercise schedule. For example, if the general fitness of the user and exercise compliance is determined to be poor, the displayed graphical representation may be coloured red and or moving slowly in the display. Similarly, if the general fitness of the user and or exercise compliance is determined to be improving, the displayed graphical representation may be coloured orange green and or moving a bit faster in the display than the displayed graphical presentation in red.

Similarly, if the general fitness of the user and/or exercise compliance is determined to be good, the displayed graphical representation may be coloured green and/or moving faster in the display.

Optionally, the graphical representation moves and or is coloured and shaped corresponding to the Body Mass Index of the user and/or compliance to the exercise schedule.

Further, according to the invention a wrist worn device is provided comprising a securing band, a control unit, a processing unit, at least one light source having a light emitting surface and at least one light receiver having a light receiving surface, an actuator arranged below each light emitting surface (e.g. below each light source), and each light receiving surface (e.g. below each light receiver), or below each combination of light emitting and light receiving surface. The at least one light source and the at least one light receiver are arranged on the securing band of the wrist worn device such that when worn the at least one light emitting surface and the at least one light receiving surface substantially abut against the palmar side of the wrist of a user. The actuator is arranged for exerting a force on the at least one light emitting surface and/or at least one light receiving surface in a direction substantially perpendicular to the palmar side of the user's wrist. The at least one light source is arranged for emitting light in a wavelength range. The at least one light receiver is arranged for producing a received light signal corresponding to a received intensity of the light received at the at least one light receiving surface. The processing unit is arranged for determining an amplitude envelope of the received light signal. The processing unit has defined therein an amplitude envelope control band having an envelope upper amplitude value and an envelope lower amplitude value. The control unit is arranged for controlling the force exerted by the actuator such that the received light signal is controlled to be within the amplitude envelope control band.

It has been found that by trying to maintain the received light signal within the amplitude envelope control band an improved received light signal is provided. This signal can be used as the basis for performing measurement and determining a health status of the user.

Optionally, the control unit is arranged for decreasing the force when the amplitude of the received light signal exceeds the envelope upper amplitude value and for increasing the force when the amplitude of the received light signal is below the envelope lower amplitude value. Optionally, the control unit is further arranged for controlling an emitted intensity of light emitted by the at least one light source such that the amplitude of the received light signal is maintained within the amplitude envelope control band.

Optionally, the wrist worn device further including a wireless communication unit arranged for communicating with a communications device, such as a smart phone, with display or a smart watch with a display.

In this way, the wrist worn device can take advantage of the smart phone's processing power, display capabilities, and user interface, e.g. via combined use with a smart phone application (app).

According to an aspect, the securing band is provided with at least one light source and at least one light receiver directly connected to the control unit on the same side of the band.

According to an aspect, the securing band is provided with at least one light source and at least one light receiver on the inner side of the wrist connected via wiring or a conductive flexfoil to the control unit which is on another point on the wrist or e.g. on the opposite, upper side of the wrist.

Optionally, the securing band is provided with woven, knitted and/or embroidered electrical connection threads connecting the at least one light source and at least one light receiver with the control unit. The securing band can be adjusted more easily to the dimensions and wrist circumferences differences of various users. Retaining the electrical connection threads in the securing band allows the control unit to be placed remotely from the at least one light emitting surface and at least one light receiving surface and allows the actuator to move without large forces the element containing the light emitting surface and light receiving surface, or the element containing the light source and receiver, and the electrical wires relatively more to the centre of the wrist or further away from the centre of the wrist. This is advantageous as the at least one light receiving surface substantially abut against the palmar side of the wrist of a user.

Optionally, the at least one light source includes an optical fiber for guiding light towards the skin of the user and/or the at least one light receiver includes an optical fiber for guiding light from the skin of the user. Advantageously, the at least one light source and/or the at least one light receiver may be remote from the skin of the user on palmar side of the wrist.

Optionally, the optical fiber is woven in, knitted in and/or embroidered on the securing band. Optionally, the optical fiber of is included in the securing band such that the ends of the fibers are mechanically touching the skin of the user. In this way the band holds the end of the fibers in position for guiding light towards and/or away from the skin of the user.

Optionally, at least a section of the optical fiber that is positioned to mechanically touch the skin of the user has been treated to allow light to couple out and/or in.

Optionally, the wrist worn device includes a plurality of actuators and a plurality of light sources and a plurality of light receivers wherein each actuator is arranged for exerting a force on one light emitting surface and/or light source and/or one light receiver and/or light receiving surface, wherein the control unit is arranged for actuating each actuator independently. Optionally, the wrist worn device includes a plurality of actuators and a plurality of light sources having a plurality of light emitting surfaces and a plurality of light receivers having a plurality of light receiving surfaces wherein each actuator is arranged for exerting a force on one light emitting surface and/or one light receiving surface, wherein the control unit is arranged for actuating each actuator independently. Optionally, the wrist worn device includes a first actuator arranged for exerting a force on a first light emitting surface and/or light source and/or light receiver and/or light receiving surface on a first side of a body part of the user, and a second actuator arranged for exerting a force on a second light emitting surface and/or light source and/or light receiver and/or light receiving surface on a second side of the body part. The first side can be different from the second side. The first side of the body part can e.g. be an upper side of the wrist. The second side of the body part can e.g. be a lower side of the wrist.

Optionally, the wrist worn device includes one or more light sources and light receivers which can be pressed on the skin more tightly via a passive spring system which is compressed slightly when the band with the light sources and light receivers on the inside is fastened to the skin. This system can be made into a controllable actuator when it's coupled to a mechanism, which tightens the band more or less based upon received light and possibly other sensor signals.

Actuating each actuator independently allows a force pressing each light emitting surface and each light receiving surface to also be controlled independently. Furthermore, independent control may be advantageous when one a single light source and light receiver is selected, or when a subset of light sources and light receivers are selected. This also applies to light emitting surfaces and light receiving surfaces. Additionally, due to the wrist of a user and the securing band the light emitting surfaces and light receiving surfaces of the device may not evenly contact the user's skin. This may be corrected for by using independently controlled actuators.

Also has been found that the effect from the ambient or surrounding light can be eliminated when the pressure increases. To guarantee the comfort of the user this pressure force can be limited via the application of a compressible and deformable light blocking shield around the light emitter and receivers and via the use of a compressible light blocking (and optionally over the light emitting and receiving surface(s) light transmitting) casing directly around the LEDs and PDs, which can move also towards or away from the arteries or centre of the wrist via a spring mechanism, a piston mechanism, a clamp or beam lever mechanism or combinations of any of the above. The modulation system for the light emitters and light receivers will also limit the influence of the environmental, ambient surrounding day light and/or artificial light. The amount of pressing, the tightness of the contact of the light emitting and receiving unit on the skin, the comfort and the desired quality of the signal can so be brought into balance to achieve an optimal product.

Optionally, the light emitting surface of the at least one light source and/or the light receiving surface of the at least one light receiver is covered with a transparent soft polymer cover and surrounded with an elastic frame, of light blocking non transparent polymer material.

In this way the light emitted from the light emitting surface is directed substantially towards the skin of the user. Stray light emitted from the light emitting surface is prevented from interfering with other emitting surfaces and from entering the light receiving surface without traveling through the user. Therefore, crosstalk between light sources and light receivers is reduced substantially. Also ambient light can be blocked by this method, combined with the mechanical force applied by the fastened securing band.

Optionally, the cover is spherically or cylindrical shaped and is arranged to be pressed inwardly of the palmar part of the wrist by adjusting the securing band and/or the actuator. A spherically shaped or cylindrical shaped cover or housing of the light emitting and light receiving surfaces improves the coupling of light emitted from the light emitting surface into the user, and improves the capture of light traveling out of the user and into the light receiving surface.

Optionally, the processing unit is arranged to detect whether the device is properly worn, or even worn at all, at the wrist on the basis of the received light signal. When the device is worn properly, an expected received light signal can be determined. On the basis of this signal or the received ambient light or combinations of both it can be determined if the received light signal corresponds to a properly worn device.

Optionally, the amount of movement and influence of artefact can be measured based on a received light signal from an addition light source with a wavelength which is different from the wavelength or wavelengths which is or which are being used to measure the desired physiological parameters. This extra wavelength can be chosen such that this light does not penetrate the skin deeply and/or has a high water absorption. This extra wavelength will more clearly show movement of the light source and the light receiver on the skin (e.g. blue and/or green LED-light, e.g. a wavelength with a high water absorption capacity of e.g. 1100-1400 nm and the corresponding sensors), measures the motion artefacts and the influence of circumferential light. The blue or green LED-lights or light emitters with a wavelength between 400 and 550 nm, or any other light emitter and receiver such as a 1100 nm system or with a higher wavelength till 1400 nm is primarily being used for the detection of movement and motion artefacts, but can if necessary also be used to perform additional heart rate or pulsation measurement in smaller blood vessels (capillaries, arterioles) directly under the skin on the upper side, inner side of left or right side of the wrist, This also applies to light emitters of other wavelengths.

Another embodiment or facility, increasing the sensitivity to the movement of the wearable device in reference to the skin, is formed by using appropriate polarisation filters at the light source(s) and the optical sensor(s). The movement sensor including a light source and an optical sensor is preferable measuring only the movement of the wearable device in reference to the skin, via the reflection of light. Therefore the movement sensor has to be predominantly sensitive to the reflection of the light on the skin and less sensitive to the scattering of light through the tissue. The light scattered through the tissue shows a pulsating element related to the pulsing of blood. This pulsating element has to be minimised in the movement sensor reading to separate the movement from the blood pulsating in the analysis of the measurements in an algorithm and software. Therefore a polarisation filter is placed on the light source. This polarisation filter can e.g. result in a light beam polarised in one direction. The light reflected on the skin rotates the polarisation direction of the light by ninety degrees. By placing a polarisation filter on the optical sensor turned by ninety degrees in reference to the polarisation filter at the light source, the sensor is more sensitive to the reflected light and less to the scattered light through the tissue. This embodiment will enable detection of heart rates under medium and more severe movement of body, arm en device on arm, due to the ability to distinguish in the signals between movement or motion artefacts and heart rate values.

Optionally, the wrist worn device further includes an accelerometer arranged for detecting the amount of movement of the wrist of the user and/or the device on the wrist of the user. This may provide an indication of the amount of everyday movement of the user. Additionally, or alternatively, this may provide an indication of a user's compliance to an exercise program or schedule. Furthermore, the output signal of the accelerometer may be provided to the processing unit and/or the control unit for taking the amount of movement of the wrist of the user into account when determining the amplitude envelope control band and/or controlling the force. Optionally, the method further comprises a way of determining the amount of physical movement or exercise of the user by a combined use and analysis of heart rate values and the detected movement via the light reflection measurement with the additional light emitter and receiver of a different wavelength, such as 400-550 nm or 1100-1400 nm, so that only a higher heart rate combined with a higher motion artefact detection indicates physical exercise. The analysis of these two measurement can be combined with data of an integrated 3-axis accelerometers in the device, a GPS receiver in the device or in a wirelessly connected communication device (smart watch, smart phone, navigation device etc.), a tri-angulation GSM positioning method in the device itself or in the wirelessly connected device, an electrical or optical temperature sensor measuring the increasing temperature of the skin during exercise.

Optionally, the processing unit is arranged for determining the blood composition of the user (haemoglobin, haematocrit value) for medical purposes such as blood deficiencies after chemo treatment and/or radiation therapy, blood loss after birth or accidents, infections with tropical diseases such as Denque, dehydration etc.

Further, according to the invention a system including a wrist worn device according to the invention including a wired or wireless communication unit and a communications device such as a display on the wrist worn device and/or on a further device, such as a smart phone, with a display connected to the system.

It will be appreciated that features described with regard to one of the method according to the invention, the wrist worn device according to the invention and the system according to the invention are considered to be disclosed for the remaining categories.

### BRIEF DESCRIPTION OF THE DRAWINGS

The invention will now be further elucidated by means of non-limiting examples referring to the drawings, in which
Fig. 1 shows a schematic top view of a wrist worn device according to the invention;
Fig. 2 shows a schematic side view of a wrist worn device according to the invention;
Fig. 3a shows an example of a measurement of a received light signal while the actuator is active;
Fig. 3b shows an example of a measurement of a received light signal while the actuator is deactivated;
Fig. 4 shows a schematic representation of a system according to the invention including a wrist worn device and a communications device;
Fig. 5 shows the system according to the invention displaying an indication of the general fitness and/or health of a user; and
Fig. 6 shows the system according to the invention displaying an indication of the general fitness and/or health of a user;
Fig. 7 shows a compressible shield around the light emitting and light receivers which blocks ambient light and allow a high comfort during wearing.

### DETAILED DESCRIPTION

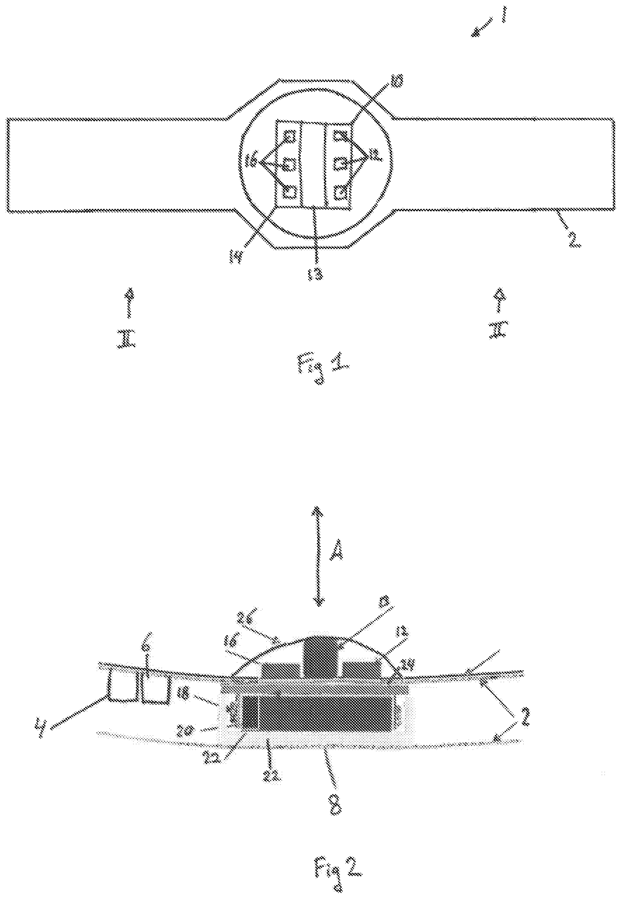

Fig. 1 shows a schematic top view of a wrist worn device 1 according to the invention, and Fig. 2 shows a schematic side view in the direction of arrows II of Fig. 1. The wrist worn device includes a securing band 2, a control unit 4, a processing unit 6, and an actuator 8. In this example, the device 1 includes three light sources each having a light emitting surface 12. In this example, the device 1 also includes three light receivers having a light receiving surface 16. The light emitting surfaces 12 and the light receiving surfaces 16 are arranged on the securing band 2 of the wrist worn device 1 such that when worn the at least one light emitting surface 12 and the at least one light receiving surface 16 substantially abut against the palmar side of the wrist of a user. Additionally in this embodiment the light emitting surfaces 12 and the light receiving surfaces 16 are separated by a non-transparent light blocker 13, which can extend to the upper surface where there will be contact with the skin. Furthermore, in this example the light emitting surfaces 12 and the light receiving surfaces 16 are received in an elastic frame 10 and 14, respectively, of light blocking material. Finally a spherically shaped transparent polymer cover 26 is provided.

In this example, the actuator 8 includes a spool 18 and a magnet 20. The spool 18 in received in a polymer or non-magnetic housing 22. The polymer housing 22 includes an actuating surface or connector 24. The actuator 8 is arranged for exerting a force on the light emitting surfaces in a direction A substantially perpendicular to the palmar side of the user's wrist. The user's wrist is not shown, however it will be clear that the surface of cover 26 of the wrist worn device 1 abuts substantially parallel to the user's wrist.

The securing band or strap can include one or more sections of stretchable textile materials and or polymer materials enabling a tight securing and delivering an initial pressing force for the actuators and light emitting and light receiving surfaces. The entire circumference of the securing band or strap can also be made of such material.

The securing band or strap can include a stretchable foam material which is permeable to water vapour, sweat and gasses, allowing ventilation of the skin. Such foam material can on the side abutting the arm or wrist be covered with a textile material allowing absorption and uptake of sweat, regulating the microclimate and preventing allergic reactions. The entire circumference of the securing band or strap can also be made of such foam material. The foam material can on the arm or wrist side be covered with a fine textile material allowing absorption and uptake of sweat, regulating the microclimate and allergic reactions.

The light receivers 16 are arranged for producing a received light signal corresponding to a received intensity of the light received at the at least one light receiving surface 16. The processing unit 6 is arranged for determining an amplitude envelope of the received light signal. The processing unit 6 has defined therein an amplitude envelope control band having an envelope upper amplitude value and an envelope lower amplitude value. The control unit 4 is arranged for controlling the force exerted by the actuator 8 such that the received light signal is controlled to be within the amplitude envelope control band. Preferably, the received light signal is maintained within the amplitude envelope control band. In this example this occurs by the control unit 4 controlling the current flow through spool, or coil, 18, which in turn creates a magnetic field and displaces the actuating surface 24 relative to the magnet 20. The displacement of the actuating surface 24 results in an increase or decrease in the pressing force parallel to direction A and, when worn, substantially perpendicular to the user's wrist.

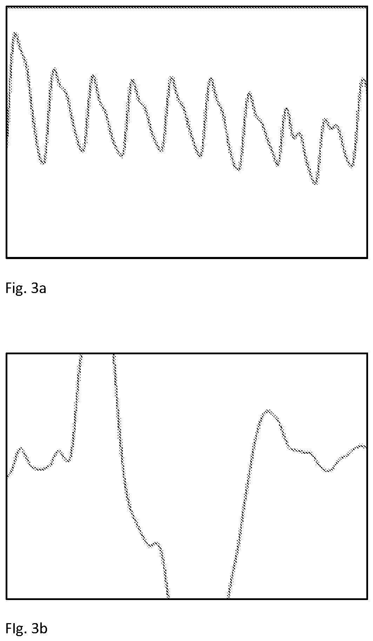

During a measurement session the amplitude of the received light signal is kept within the amplitude envelope control band. Measurements derived from a received light signal where the amplitude of the received light signal has been kept within the amplitude envelope control band during a measurement session are more reliable. In this example, a user's heart rate and heart rate variability are determined on the basis of the received light signal. Figure 3a shows an example of a measurement of the received light signal as a function of time while the actuator 8 is active to control the signal to be within the amplitude envelope control band. The heart beats are clearly visible in Fig. 3a. Figure 3b shows an example of a measurement of the received light signal as a function of time while the actuator 8 is deactivated. The difference with Fig. 3a is big, and all individual heartbeats cannot be identified in the measured signal in Fig. 3b, which is disturbed largely by the motion artefacts (movement of light emitters and receivers on skin, movement of limbs, flow of blood).

Additionally, in this example the three light emitting sources are each capable of emitting light in a first wavelength range and a second wavelength range. In this example, each light source comprises a first LED capable of emitting light in a first wavelength range of 600-660 nm and a second LED capable of emitting light in a second wavelength range of 880-940 nm, or in wavelengths, which are capable of detection of other gases, solids or liquids in the blood (e.g. 700-900 nm for CO₂)

By performing a first measurement session wherein the light emitted by the three light sources in the first wavelength range and performing a second measurement session wherein the light emitted by the three light sources is in the second wavelength range additional health indicators such as the user's saturation of peripheral oxygen level, SpO2, can be determined. This is determined on the basis of a ratio of the perfusion index value calculated in the first measurement session and the perfusion index value calculated in the second measurement session.

In this example, prior to performing a measurement session a selection step is performed. In the selection step a light source and light receiver combination is selected. For each light source of the three light sources, light is emitted in a wavelength range for a predetermined amount of time. A received light signal is produced at each of the light receivers. The processing unit 6 separates each received light signal into a high frequency component and a low frequency component using filters with cut-off frequency values that are based on the lowest expected heart rate and the highest expected heart rate. A perfusion index value is calculated for each received light signal by the processing unit 6 on the basis of a ratio of the high frequency component and the low frequency component. The steps are repeated for each light source, and the light source and light receiver combination having the highest perfusion index value is selected. It is possible that after this selection the non-selected light sources are switched off to reduce power consumption.

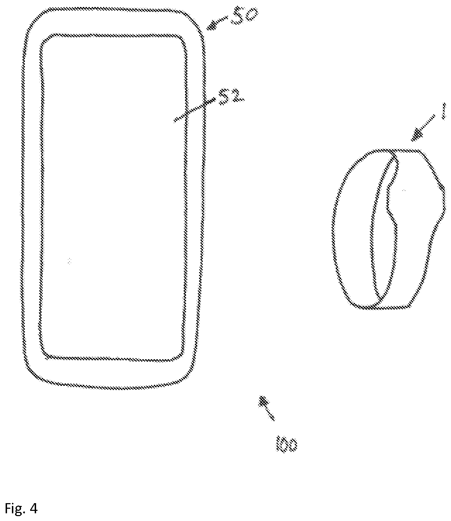

A system 100 including a wrist worn device 1 and a communications device 50 is shown in Fig. 4. The communications device 50 includes a display, e.g. a touch screen display, 52, which gives information, feedback and instructions to the user and which software application can also be used to define, start and install measurement sessions. In this example, the display 52 is associated with the wrist worn device. Additionally both the communications device 50 and the wrist worn device 1 include a communication unit, not pictured, through which the devices are able to communicate with each other. In this example the communications and controlling device 50 can for instance be a smart phone device with a touch screen display.

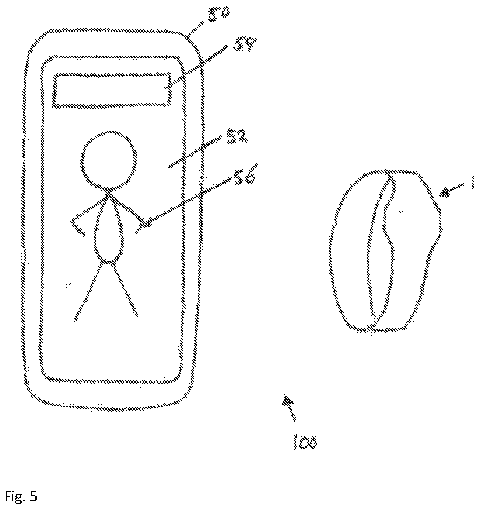

With the system 100, a user can monitor his general fitness. In addition, a user and/or a health advisor can monitor a user's compliance to a selected exercise schedule. For example, in Fig. 5, an exercise instruction 54 appears on the display 52 of the communications device 50. For example, the instruction 54 instructs the user to perform e.g. twenty knee bendings (so called squats). During the exercise a measurement session is performed. Preferably, a measurement session is performed prior to starting the exercise and, e.g. at predefined intervals, after the exercise is completed. It is possible that the wrist worn device 1 concludes that the exercise is completed on the basis of at least one of an amount of movement determined on the basis of the received light signal, an amount of movement determined by an accelerometer of the wrist worn device, and a measured elapsed time interval.

In this case during each measurement session the user's heart rate, heart rate variability, and SpO2 is measured. On the basis of these measurements recorded during different measurement sessions, an indication of a user's general fitness and/or indication of the user's health is determined.

On the display 52 of communications device 50 associated with the wrist worn device 1, an indication 56 of the general fitness of the user is displayed. The indication 56 may relate to the general fitness just measured during the exercise. Additionally, or alternatively, the indication 56 may relate to a general fitness measured on the basis of the measurement session and one or more previous measurement sessions, for example measurement sessions taken over the previous week or month.

In this example, the indication 56 is a graphical representation of a person having a general fitness corresponding to the general fitness of the user or an activity pattern or movement achievement during a certain amount of time. In Fig. 5, the measured general fitness of the user is poor. Therefore a graphical representation 56 of a person having a poor general fitness is shown. In this example, the graphical representation 56 is of an overweight person. Additionally, in the case that the graphical representation 56 of the person is animated, the animation can represent the general fitness of the user, e.g. graphical representation 56 of the person can be animated to be breathing heavily.

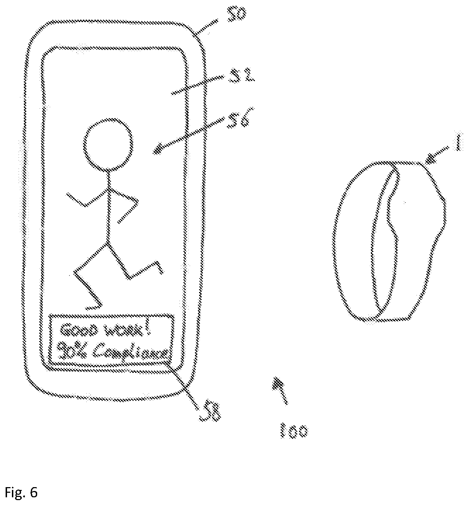

In Fig. 6, the measured general fitness of the user is good. Therefore a graphical representation 56 of a person having a good general fitness is shown. The graphical representation 56 is a of healthy person of normal build. Additionally, in the case that the graphical representation 56 of the person is animated, the graphical representation 56 of the person can e.g. be moving fast or running in place.

The indication 56 of a general fitness of the user can also be measured on the basis of measurement session initiated in response to an amount of movement being detected by the accelerometers provided, not pictured, in the wrist worn device. The indication 56 of a general fitness of the user can also be measured on the basis of measurement session initiated in response to an amount of movement being detected by the integrated optical motion artefact and movement detection system, and if necessary with additional sensors such as provided accelerometers, not pictured, in the wrist worn device, or GPS positioning data and/or GSM tri-angulation data. The amount of movement and the measured health status are recorded in the wrist worn device 1 and/or the communications device 50. In this way a measure of compliance to an exercise schedule is measured. For example, the schedule might require that the user performs 30 minutes of exercise a day at or above the users target heart rate. In the case of Fig. 6, the user has achieved a 90 % compliance. In the example of Fig. 6 this is displayed as a text 58, including an encouraging comment, on display 52 of the communications device 50 or a changing colour of the graphical presentation of a person or higher moving speed of the graphical presentation of a person.

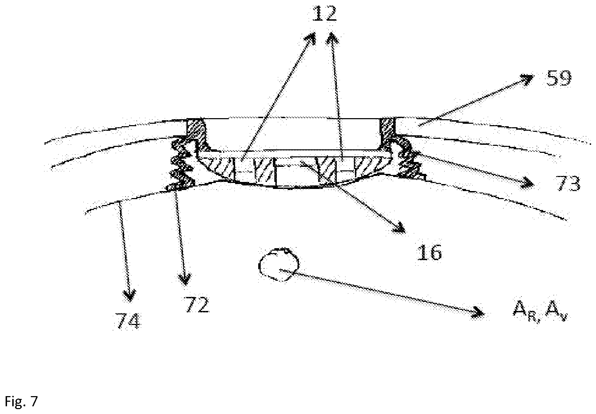

Fig. 7. shows a compressible non-transparent shield 73 around the light emitting surfaces 12 and light receiving surfaces and 16 which blocks ambient light and allow a high comfort during wearing. The shield is deformable due to its shape and/or use of flexible, compressible material. The shield 73 has ends 72 which can rest upon the skin and block all ambient light from the environment. The shield can have a bellows shape or another deformable shape allowing compression after applying force on the fastening strap, or via another method.

In the foregoing specification, the invention has been described with reference to specific examples of embodiments of the invention. It will, however, be evident that various modifications and changes may be made therein without departing from the scope of the invention as set forth in the appended claims.

However, other modifications, variations, and alternatives are also possible. The specifications, drawings and examples are, accordingly, to be regarded in an illustrative rather than in a restrictive sense.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to advantage.

## Claims

1. Method for measuring a signal representative of a physiological parameter of a moving user comprising the steps of:
providing a device (1) worn on a body of the user, wherein the device includes, a securing band (2), at least one sensor (16) for measuring the signal representative of the physiological parameter; wherein the at least one sensor is arranged on the securing band of the device such that when worn, the at least one sensor abuts against the body of the user; and
performing a measurement session including;
measuring a value of the signal using the at least one sensor;
defining an amplitude envelope control band having an envelope upper amplitude value and an envelope lower amplitude value by measuring the signal during a predetermined period, and determining the envelope upper amplitude value and envelope lower amplitude value on the basis of the measured value during the predetermined period, and
controlling the amplitude of the measured value to be within the amplitude envelope control band by controlling a force pressing the at least one sensor in a direction substantially perpendicular to the body of the user,
wherein the step of determining the amplitude envelope control band is repeated at moments during the measuring session so as to set a new amplitude envelope control band during the measurement session.

2. Method according to claim 1, wherein the controlling includes decreasing the force when the amplitude of the received signal exceeds the envelope upper amplitude value and increasing the force when the amplitude of the received light signal is below the envelope lower amplitude value.

3. Method according to claim 1 or 2, wherein the sensor includes a light source having a light emitting surface and at least one light receiver having a light receiving surface; wherein the at least one light source and the at least one light receiver are arranged on the securing band of the device such that when worn, the at least one light emitting surface and the at least one light receiving surface substantially abut against the palmar side of the wrist of the user; and
wherein the step of measuring the signal includes:
emitting light in a wavelength range by the at least one light source for a predetermined amount of time;
producing a received light signal by the at least one light receiver corresponding to a received intensity of light received at the at least one light receiver; and
wherein the step of controlling includes controlling the amplitude of the received light signal to be within the amplitude envelope control band by controlling a force pressing the at least one light emitting surface and/or at least one light receiving surface in a direction substantially perpendicular to the palmar side of the user's wrist, optionally further including controlling the amplitude of the received light signal to be within the amplitude envelope control band by controlling an emitted intensity of light emitted by the at least one light source.

4. Method according to any one of the preceding claims, wherein the step of determining the amplitude envelope control band is performed continuously during the measurement session.

5. Method according to any one of the preceding claims, further including determining a relationship between the pressing force and the measured value, optionally including applying a step in the force pressing the at least one sensor in a direction substantially perpendicular to the user's body and determining a step response of the measured signal,
wherein optionally the relationship between the pressing force and the measured signal value is determined for a range of forces exerted on the at least sensor, the method optionally further including storing the relationship.

6. Method according to any one of the preceding claims as far as dependent on claim 3, further including determining the user's heart rate on the basis of the received light signal based on time and/or frequency domain analysis, and optionally on the basis of a value of a control signal of the control unit for controlling the force and/or emitted intensity.

7. Method according to any one of the preceding claims as far as dependent on claim 3, further including performing a first measurement session wherein the light emitted by the at least one light source is in a first wavelength range and performing a second measurement session wherein the light emitted by the at least one light source is in a second wavelength range, wherein optionally the step of performing a measurement session further includes a step of calculating a perfusion index value on the basis of the received light signal; and
wherein the method optionally further includes determining the user's saturation of peripheral oxygen level on the basis of a ratio of the perfusion index value calculated in the first measurement session and the perfusion index value calculated in the second measurement session, and optionally on the basis of a value of a control signal of the control unit for controlling the force and/or emitted intensity.

8. Method according to any one of the preceding claims, wherein the actuator includes one of a spool and magnet, and an inflatable body, a piezoelectric actuator, a linear motor, a gear, a conductive polymer or textile structure able to alter it's dimensions in radial, length and/or width directions when a current is applied.

9. Method according to any one of the preceding claims, wherein the physiological parameter is at least one of heart rate, heart rate variability, heart rate recovery rate, respiration rate, CO2-level in blood vessels, oxygen saturation (SpO2-level), movement and/or activity.

10. Body worn device (1) for measuring a signal representative of a physiological parameter comprising a securing band (2), a control unit (4), a processing unit (6), an actuator (8), and at least one sensor (16) for measuring the signal value; wherein the at least one sensor is arranged on the securing band of the body worn device such that when worn the at least one sensor abuts against the body of a user;
wherein the actuator is arranged for exerting a force on the at least one sensor in a direction substantially perpendicular to the body of the user;
wherein the processing unit is arranged for determining, and has defined therein, an amplitude envelope control band having an envelope upper amplitude value and an envelope lower amplitude value by measuring the signal during a predetermined period, and determining the envelope upper amplitude value and envelope lower amplitude value on the basis of the measured value during the predetermined period, and
wherein the control unit is arranged for controlling the force exerted by the actuator such that the measured signal value is within the amplitude envelope control band,
wherein the processing unit is further arranged for repeating the determining of the amplitude envelope control band at moments during a measuring session so as to set a new amplitude envelope control band during the measurement session.

11. Device according to claim 10, wherein the control unit is arranged for decreasing the force when the amplitude of the measured signal exceeds the envelope upper amplitude value and for increasing the force when the amplitude of the measured signal is below the envelope lower amplitude value.

12. Device according to claim 10 or 11 wherein the at least one sensor includes at least one light source having a light emitting surface and at least one light receiver having a light receiving surface; wherein the at least one light source and the at least one light receiver are arranged on the securing band of the device such that when worn the at least one light emitting surface and the at least one light receiving surface substantially abut against the palmar side of the wrist of a user;
wherein the actuator is arranged for exerting a force on the at least one light source and/or at least one light receiver in a direction substantially perpendicular to the palmar side of the user's wrist;
wherein the at least one light source is arranged for emitting light in a wavelength range;
wherein the at least one light receiver is arranged for producing a received light signal corresponding to a received intensity of the light received at the at least one light receiving surface;
wherein the processing unit is arranged for determining an amplitude envelope of the received light signal;
wherein the processing unit has defined therein an amplitude envelope control band having an envelope upper amplitude value and an envelope lower amplitude value, and
wherein the control unit is arranged for controlling the force exerted by the actuator such that the received light signal is within the amplitude envelope control band.

13. Device according to claim 12, wherein the control unit is further arranged for controlling an emitted intensity of light emitted by the at least one light source such that the amplitude of the received light signal is within the amplitude envelope control band.

14. Device according to any one claims 10-13, further including a wireless communication unit arranged for communicating with a communications device, such as smart phone with display.

15. Device according to claim 12 or any one of claims 13-14 as far as dependent on claim 12, wherein the at least one light source includes an optical fiber for guiding light towards the skin of the user and/or wherein the at least one light receiver includes an optical fiber for guiding light from the skin of the user,
wherein optionally at least a section of the optical fiber positioned to mechanically touch the skin of the user has been treated to allow light to couple out and/or in.

## Patentansprüche

1. Verfahren zum Messen eines Signals, das repräsentativ für einen physiologischen Parameter eines sich bewegenden Benutzers ist und die folgenden Schritte umfasst:
Bereitstellen einer an einem Körper des Benutzers getragenen Vorrichtung (1), wobei die Vorrichtung ein Befestigungsband (2), wenigstens einen Sensor (16) zum Messen des für den physiologischen Parameter repräsentativen Signals einschließt; wobei der wenigstens eine Sensor am Befestigungsband der Vorrichtung angeordnet ist, so dass der wenigstens eine Sensor beim Tragen am Körper des Benutzers anliegt; und
Durchführen einer Messsitzung, die einschließt:
Messen eines Wertes des Signals unter Verwendung des wenigstens einen Sensors;
Definieren eines Regelungsbands der Amplitudenhüllkurve mit einem oberen Amplitudenwert der Hüllkurve und einem unteren Amplitudenwert der Hüllkurve durch Messen des Signals während eines vorbestimmten Zeitraums und Bestimmen des oberen Amplitudenwerts der Hüllkurve und des unteren Amplitudenwerts der Hüllkurve auf der Grundlage des Messwerts während des vorbestimmten Zeitraums und
Regelung der Amplitude des Messwerts innerhalb des Regelungsbands der Amplitudenhüllkurve durch Steuerung einer Kraft, die den wenigstens einen Sensor in einer Richtung im Wesentlichen senkrecht zum Körper des Benutzers drückt,
wobei der Schritt des Bestimmens des Regelungsbands der Amplitudenhüllkurve zu bestimmten Zeitpunkten während der Messsitzung wiederholt wird, um während der Messsitzung ein neues Regelungsband der Amplitudenhüllkurve einzustellen.

2. Verfahren nach Anspruch 1, wobei die Regelung das Verringern der Kraft, wenn die Amplitude des empfangenen Signals den oberen Amplitudenwert der Hüllkurve überschreitet, und das Erhöhen der Kraft, wenn die Amplitude des empfangenen Lichtsignals unter dem unteren Amplitudenwert der Hüllkurve liegt, einschließt.

3. Verfahren nach Anspruch 1 oder 2, wobei der Sensor eine Lichtquelle mit einer lichtemittierenden Oberfläche und wenigstens einen Lichtempfänger mit einer lichtempfangenden Oberfläche aufweist; wobei die wenigstens eine Lichtquelle und der wenigstens eine Lichtempfänger auf dem Befestigungsband der Vorrichtung angeordnet sind, so dass die wenigstens eine lichtemittierende Oberfläche und die wenigstens eine lichtempfangende Oberfläche beim Tragen im Wesentlichen an der Handflächenseite des Handgelenks des Benutzers anliegen; und
wobei der Schritt des Messens des Signals einschließt:
Emittieren von Licht in einem Wellenlängenbereich durch die wenigstens eine Lichtquelle für eine vorbestimmte Zeitspanne;
Erzeugen eines empfangenen Lichtsignals durch den wenigstens einen Lichtempfänger entsprechend einer empfangenen Lichtintensität, die an dem wenigstens einen Lichtempfänger empfangen wird; und
wobei der Schritt der Steuerung die Regelung der Amplitude des empfangenen Lichtsignals einschließt, um innerhalb des Regelungsbands der Amplitudenhüllkurve zu liegen, indem eine Kraft gesteuert wird, die die wenigstens eine lichtemittierende Oberfläche und/oder wenigstens eine lichtempfangende Oberfläche in einer Richtung im Wesentlichen senkrecht zur Handflächenseite des Handgelenks des Benutzers drückt, und optional ferner die Regelung der Amplitude des empfangenen Lichtsignals, um innerhalb des Steuerbands der Amplitudenhüllkurve zu liegen, indem eine emittierte Lichtintensität, die von der wenigstens einen Lichtquelle emittiert wird, geregelt wird.

4. Verfahren nach einem der vorhergehenden Ansprüche, wobei der Schritt des Bestimmens des Regelungsbands der Amplitudenhüllkurve während der Messsitzung kontinuierlich durchgeführt wird.

5. Verfahren nach einem der vorhergehenden Ansprüche, das ferner das Bestimmen eines Zusammenhangs zwischen der Druckkraft und dem Messwert einschließt, das optional das Anwenden eines Schritts in der Kraft einschließt, der den wenigstens einen Sensor in einer Richtung im Wesentlichen senkrecht zum Körper des Benutzers drückt, und das Bestimmen einer Schrittantwort des gemessenen Signals,
wobei das Verhältnis optional zwischen der Druckkraft und dem gemessenen Signalwert für einen Bereich von Kräften bestimmt wird, die auf den wenigstens Sensor ausgeübt werden, wobei das Verfahren optional ferner Speichern des Verhältnisses einschließt.

6. Verfahren nach einem der vorhergehenden Ansprüche, soweit es von Anspruch 3 abhängt, das ferner das Bestimmen der Herzfrequenz des Benutzers auf der Grundlage des empfangenen Lichtsignals basierend auf einer Zeit- und/oder Frequenzbereichsanalyse und optional auf der Grundlage eines Werts eines Steuersignals der Steuereinheit zur Steuerung der Kraft und/oder der emittierten Intensität einschließt.

7. Verfahren nach einem der vorhergehenden Ansprüche, soweit es von Anspruch 3 abhängt, das ferner das Durchführen einer ersten Messsitzung einschließt, wobei das von der wenigstens einen Lichtquelle emittierte Licht in einem ersten Wellenlängenbereich liegt, und das Durchführen einer zweiten Messsitzung, wobei das von der wenigstens einen Lichtquelle emittierte Licht in einem zweiten Wellenlängenbereich liegt, wobei optional der Schritt des Durchführens einer Messsitzung ferner einen Schritt der Berechnung eines Perfusionsindexwertes auf der Grundlage des empfangenen Lichtsignals umfasst; und
wobei das Verfahren ferner optional das Bestimmen der Sättigung des Benutzers an peripherem Sauerstoffniveau auf der Grundlage eines Verhältnisses des in der ersten Messsitzung berechneten Perfusionsindexwertes und des in der zweiten Messsitzung berechneten Perfusionsindexwertes und optional auf der Grundlage eines Wertes eines Steuersignals der Steuereinheit zur Steuerung der Kraft und/oder emittierten Intensität einschließt.

8. Verfahren nach einem der vorhergehenden Ansprüche, wobei der Aktuator eine Spule und einen Magneten und einen aufblasbaren Körper, einen piezoelektrischen Aktuator, einen Linearmotor, ein Getriebe, ein leitfähiges Polymer oder eine Textilstruktur einschließt, die in der Lage sind, ihre Abmessungen in Radial-, Längen- und/oder Breitenrichtung zu ändern, wenn ein Strom angelegt wird.

9. Verfahren nach einem der vorhergehenden Ansprüche, wobei der physiologische Parameter wenigstens eine von Herzfrequenz, Herzfrequenzvariabilität, Herzfrequenz-Erholungsrate, Atemfrequenz, CO2-Gehalt in Blutgefäßen, Sauerstoffsättigung (SpO2-Gehalt), Bewegung und/oder Aktivität ist.

10. Am Körper getragene Vorrichtung (1) zum Messen eines Signals, das für einen physiologischen Parameter repräsentativ ist, mit einem Befestigungsband (2), einer Steuereinheit (4), einer Verarbeitungseinheit (6), einem Aktuator (8) und wenigstens einem Sensor (16) zum Messen des Signalwerts; wobei der wenigstens eine Sensor an dem Befestigungsband der am Körper getragenen Vorrichtung angeordnet ist, so dass der wenigstens eine Sensor beim Tragen an dem Körper eines Benutzers anliegt;
wobei der Aktuator zur Ausübung einer Kraft auf den wenigstens einen Sensor in einer Richtung im Wesentlichen senkrecht zum Körper des Benutzers angeordnet ist;
wobei die Verarbeitungseinheit zum Bestimmen eines Regelungsbands der Amplitudenhüllkurve mit einem oberen Amplitudenwert der Hüllkurve und einem unteren Amplitudenwert der Hüllkurve durch Messen des Signals während eines vorbestimmten Zeitraums und Bestimmen des oberen Amplitudenwerts der Hüllkurve und des unteren Amplitudenwerts der Hüllkurve auf der Grundlage des Messwerts während des vorbestimmten Zeitraums angeordnet ist und diese darin definiert hat, und
wobei die Steuereinheit zur Steuerung der vom Aktuator ausgeübten Kraft angeordnet ist, so dass der gemessene Signalwert innerhalb des Regelungsbands der Amplitudenhüllkurve liegt,
wobei die Verarbeitungseinheit ferner zum Wiederholen des Bestimmens des Regelungsbands der Amplitudenhüllkurve zu bestimmten Zeitpunkten während einer Messsitzung angeordnet ist, um während der Messsitzung ein neues Regelungsband der Amplitudenhüllkurve einzustellen.

11. Vorrichtung nach Anspruch 10, wobei die Steuereinheit zum Verringern der Kraft, wenn die Amplitude des gemessenen Signals den oberen Amplitudenwert der Hüllkurve überschreitet, und zum Erhöhen der Kraft, wenn die Amplitude des gemessenen Signals unter dem unteren Amplitudenwert der Hüllkurve liegt, ausgebildet ist.

12. Vorrichtung nach Anspruch 10 oder 11, wobei der wenigstens eine Sensor wenigstens eine Lichtquelle mit einer lichtemittierenden Oberfläche und wenigstens einen Lichtempfänger mit einer lichtemittierenden Oberfläche einschließt; wobei die wenigstens eine Lichtquelle und der wenigstens eine Lichtempfänger auf dem Befestigungsband der Vorrichtung angeordnet sind, so dass, beim Tragen die wenigstens eine lichtemittierende Oberfläche und die wenigstens eine lichtempfangende Oberfläche im Wesentlichen an der Handflächenseite des Handgelenks eines Benutzers anliegen;
wobei der Aktuator zur Ausübung einer Kraft auf die wenigstens eine Lichtquelle und/oder wenigstens einen Lichtempfänger in einer Richtung im Wesentlichen senkrecht zur Handflächenseite des Handgelenks des Benutzers angeordnet ist;
wobei die wenigstens eine Lichtquelle zum Emittieren von Licht in einem Wellenlängenbereich angeordnet ist;
wobei der wenigstens eine Lichtempfänger zum Erzeugen eines empfangenen Lichtsignals entsprechend einer empfangenen Intensität des an der wenigstens einen lichtempfangenden Oberfläche empfangenen Lichts angeordnet ist;
wobei die Verarbeitungseinheit zum Bestimmen einer Amplitudenhüllkurve des empfangenen Lichtsignals angeordnet ist;
wobei die Verarbeitungseinheit darin ein Regelungsband für die Amplitudenhüllkurve mit einem oberen Amplitudenwert der Hüllkurve und einem unteren Amplitudenwert der Hüllkurve definiert hat, und
wobei die Steuereinheit zur Steuerung der vom Aktuator ausgeübten Kraft angeordnet ist, so dass das empfangene Lichtsignal innerhalb des Regelungsbands der Amplitudenhüllkurve liegt.

13. Vorrichtung nach Anspruch 12, wobei die Steuereinheit ferner zur Steuerung einer emittierten Lichtintensität, die von der wenigstens einen Lichtquelle emittiert wird, ausgebildet ist, so dass die Amplitude des empfangenen Lichtsignals innerhalb des Steuerbands der Amplitudenhüllkurve liegt.

14. Vorrichtung nach einem der Ansprüche 10 bis 13, die ferner eine drahtlose Kommunikationseinheit einschließt, die zur Kommunikation mit einer Kommunikationsvorrichtung, wie einem Smartphone mit Display, angeordnet ist.

15. Vorrichtung nach Anspruch 12 oder einem der Ansprüche 13 bis 14, soweit sie von Anspruch 12 abhängt, wobei die wenigstens eine Lichtquelle eine optische Faser zum Leiten von Licht zu der Haut des Benutzers umfasst und/oder wobei der wenigstens eine Lichtempfänger eine optische Faser zum Leiten von Licht von der Haut des Benutzers einschließt, wobei optional wenigstens ein Abschnitt der optischen Faser, der so positioniert ist, dass er die Haut des Anwenders mechanisch berührt, so behandelt wurde, dass er das Ein- und/oder Auskoppeln von Licht ermöglicht.

## Revendications

1. Procédé pour mesurer un signal représentatif d'un paramètre physiologique d'un utilisateur en mouvement comprenant les étapes consistant à :
fournir un dispositif (1) porté sur un corps de l'utilisateur, dans lequel le dispositif comporte
une bande de fixation (2), au moins un capteur (16) pour mesurer le signal représentatif du paramètre physiologique ; l'au moins un capteur étant agencé sur la bande de fixation du dispositif de sorte que lorsque celui-ci est porté, l'au moins un capteur vienne en butée contre le corps de l'utilisateur ; et
réaliser une session de mesure comportant
la mesure d'une valeur du signal en utilisant l'au moins un capteur ;
la définition d'une bande de commande d'enveloppe d'amplitude ayant une valeur d'amplitude supérieure d'enveloppe et une valeur d'amplitude inférieure d'enveloppe en mesurant le signal pendant une période prédéterminée, et la détermination de la valeur d'amplitude supérieure d'enveloppe et de la valeur d'amplitude inférieure d'enveloppe sur la base de la valeur mesurée pendant la période prédéterminée, et
la commande de l'amplitude de la valeur mesurée pour qu'elle soit dans la bande de commande d'enveloppe d'amplitude en commandant une force pressant l'au moins un capteur dans une direction sensiblement perpendiculaire au corps de l'utilisateur,
dans lequel l'étape de détermination de la bande de commande d'enveloppe d'amplitude est répétée à des moments pendant la session de mesure de manière à définir une nouvelle bande de commande d'enveloppe d'amplitude pendant la session de mesure.

2. Procédé selon la revendication 1, dans lequel la commande comporte la diminution de la force lorsque l'amplitude du signal reçu dépasse la valeur d'amplitude supérieure d'enveloppe, et l'augmentation de la force lorsque l'amplitude du signal lumineux reçu est en dessous de la valeur d'amplitude inférieure d'enveloppe.

3. Procédé selon la revendication 1 ou 2, dans lequel le capteur comporte une source de lumière ayant une surface électroluminescente et au moins un récepteur de lumière ayant une surface de réception de lumière ; dans lequel l'au moins une source de lumière et l'au moins un récepteur de lumière sont agencés sur la bande de fixation du dispositif de sorte que, lorsque celui-ci est porté, l'au moins une surface électroluminescente et l'au moins une surface de réception de lumière viennent sensiblement en butée contre le côté palmaire du poignet de l'utilisateur ; et
dans lequel l'étape de mesure du signal comporte :
l'émission d'une lumière dans une plage de longueurs d'onde par l'au moins une source de lumière pendant une durée prédéterminée ;
la production d'un signal lumineux reçu par l'au moins un récepteur de lumière correspondant à une intensité de lumière reçue, reçue au niveau de l'au moins un récepteur de lumière ; et
dans lequel l'étape de commande comporte la commande de l'amplitude du signal lumineux reçu pour qu'elle soit dans la bande de commande d'enveloppe d'amplitude en commandant une force pressant l'au moins une surface électroluminescente et/ou l'au moins une surface de réception de lumière dans une direction sensiblement perpendiculaire au côté palmaire du poignet de l'utilisateur, facultativement, comportant en outre la commande de l'amplitude du signal lumineux reçu pour qu'elle soit dans la bande de commande d'enveloppe d'amplitude en commandant une intensité de lumière émise, émise par l'au moins une source de lumière.

4. Procédé selon l'une quelconque des revendications précédentes, dans lequel l'étape de détermination de la bande de commande d'enveloppe d'amplitude est réalisée en continu pendant la session de mesure.

5. Procédé selon l'une quelconque des revendications précédentes, comportant en outre la détermination d'une relation entre la force de pression et la valeur mesurée, facultativement, comportant l'application d'une étape dans la force pressant l'au moins un capteur dans une direction sensiblement perpendiculaire au corps de l'utilisateur et la détermination d'une réponse d'étape du signal mesuré,
dans lequel, facultativement, la relation entre la force de pression et la valeur de signal mesuré est déterminée pour une plage de forces exercées sur l'au moins un capteur, le procédé comportant facultativement en outre le stockage de la relation.

6. Procédé selon l'une quelconque des revendications précédentes dans la mesure où elles dépendent de la revendication 3, comportant en outre la détermination de la fréquence cardiaque de l'utilisateur sur la base du signal lumineux reçu sur la base d'une analyse dans le domaine temporel et/ou fréquentiel, et, facultativement, sur la base d'une valeur d'un signal de commande de l'unité de commande pour commander la force et/ou l'intensité émise.

7. Procédé selon l'une quelconque des revendications précédentes dans la mesure où elles dépendent de la revendication 3, comportant en outre la réalisation d'une première session de mesure où la lumière émise par l'au moins une source de lumière est dans une première plage de longueurs d'onde, et la réalisation d'une deuxième session de mesure où la lumière émise par l'au moins une source de lumière est dans une deuxième plage de longueurs d'onde, dans lequel, facultativement, l'étape de réalisation d'une session de mesure comporte en outre une étape de calcul d'une valeur d'indice de perfusion sur la base du signal lumineux reçu ; et
dans lequel le procédé comporte facultativement en outre la détermination du niveau de saturation en oxygène périphérique de l'utilisateur sur la base d'un rapport de la valeur d'indice de perfusion calculée dans la première session de mesure et de la valeur d'indice de perfusion calculée dans la deuxième session de mesure, et, facultativement, sur la base d'une valeur d'un signal de commande de l'unité de commande pour commander la force et/ou l'intensité émise.

8. Procédé selon l'une quelconque des revendications précédentes, dans lequel l'actionneur comporte l'un parmi une bobine et un aimant, et un corps gonflable, un actionneur piézoélectrique, un moteur linéaire, un engrenage, un polymère conducteur ou une structure textile en mesure de modifier ses dimensions dans des directions radiale, longitudinale et/ou transversale lorsqu'un courant est appliqué.

9. Procédé selon l'une quelconque des revendications précédentes, dans lequel le paramètre physiologique est au moins l'un parmi une fréquence cardiaque, une variabilité de fréquence cardiaque, un taux de récupération de fréquence cardiaque, une fréquence respiratoire, un niveau de CO2 dans les vaisseaux sanguins, une saturation en oxygène (niveau SpO2), un déplacement et/ou une activité.

10. Dispositif porté sur le corps (1) pour mesurer un signal représentatif d'un paramètre physiologique comprenant une bande de fixation (2), une unité de commande (4), une unité de traitement (6), un actionneur (8), et au moins un capteur (16) pour mesurer la valeur de signal ; dans lequel l'au moins un capteur est agencé sur la bande de fixation du dispositif porté sur le corps de sorte que lorsque celui-ci est porté, l'au moins un capteur vient en butée contre le corps d'un utilisateur ;
dans lequel l'actionneur est agencé pour exercer une force sur l'au moins un capteur dans une direction sensiblement perpendiculaire au corps de l'utilisateur ;
dans lequel l'unité de traitement est agencée pour déterminer, et a défini dans celle-ci, une bande de commande d'enveloppe d'amplitude ayant une valeur d'amplitude supérieure d'enveloppe et une valeur d'amplitude inférieure d'enveloppe en mesurant le signal pendant une période prédéterminée, et déterminer la valeur d'amplitude supérieure d'enveloppe et la valeur d'amplitude inférieure d'enveloppe sur la base de la valeur mesurée pendant la période prédéterminée, et
dans lequel l'unité de commande est agencée pour commander la force exercée par l'actionneur de sorte que la valeur de signal mesuré soit dans la bande de commande d'enveloppe d'amplitude,
dans lequel l'unité de traitement est en outre agencée pour répéter la détermination de la bande de commande d'enveloppe d'amplitude à des moments pendant une session de mesure de manière à définir une nouvelle bande de commande d'enveloppe d'amplitude pendant la session de mesure.

11. Dispositif selon la revendication 10, dans lequel l'unité de commande est agencée pour diminuer la force lorsque l'amplitude du signal mesuré dépasse la valeur d'amplitude supérieure d'enveloppe et pour augmenter la force lorsque l'amplitude du signal mesuré est en dessous de la valeur d'amplitude inférieure d'enveloppe.

12. Dispositif selon la revendication 10 ou 11 dans lequel l'au moins un capteur comporte au moins une source de lumière ayant une surface électroluminescente et au moins un récepteur de lumière ayant une surface de réception de lumière ; dans lequel l'au moins une source de lumière et l'au moins un récepteur de lumière sont agencés sur la bande de fixation du dispositif de sorte que lorsque celui-ci est porté, l'au moins une surface électroluminescente et l'au moins une surface de réception de lumière viennent sensiblement en butée contre le côté palmaire du poignet d'un utilisateur ;
dans lequel l'actionneur est agencé pour exercer une force sur l'au moins une source de lumière et/ou au moins un récepteur de lumière dans une direction sensiblement perpendiculaire au côté palmaire du poignet de l'utilisateur ;
dans lequel l'au moins une source de lumière est agencée pour émettre une lumière dans une plage de longueurs d'onde ;
dans lequel l'au moins un récepteur de lumière est agencé pour produire un signal lumineux reçu correspondant à une intensité reçue de la lumière reçue au niveau de l'au moins une surface de réception de lumière ;
dans lequel l'unité de traitement est agencée pour déterminer une enveloppe d'amplitude du signal lumineux reçu ;
dans lequel l'unité de traitement a défini dans celle-ci une bande de commande d'enveloppe d'amplitude ayant une valeur d'amplitude supérieure d'enveloppe et une valeur d'amplitude inférieure d'enveloppe, et
dans lequel l'unité de commande est agencée pour commander la force exercée par l'actionneur de sorte que le signal lumineux reçu soit dans la bande de commande d'enveloppe d'amplitude.

13. Dispositif selon la revendication 12, dans lequel l'unité de commande est en outre agencée pour commander une intensité de lumière émise, émise par l'au moins une source de lumière de sorte que l'amplitude du signal lumineux reçu soit dans la bande de commande d'enveloppe d'amplitude.

14. Dispositif selon l'une quelconque des revendications 10 à 13, comportant en outre une unité de communication sans fil agencée pour communiquer avec un dispositif de communication, tel qu'un téléphone intelligent avec écran.

15. Dispositif selon la revendication 12 ou l'une quelconque des revendications 13 et 14 dans la mesure où elles dépendent de la revendication 12, dans lequel l'au moins une source de lumière comporte une fibre optique pour guider une lumière vers la peau de l'utilisateur et/ou dans lequel l'au moins un récepteur de lumière comporte une fibre optique pour guider une lumière à partir de la peau de l'utilisateur,
dans lequel, facultativement, au moins une section de la fibre optique positionnée pour toucher mécaniquement la peau de l'utilisateur a été traitée pour permettre à la lumière de sortir et/ou d'entrer.
